# EUROPEAN PATENT APPLICATION

(11) **EP 3 960 105 A1**
(43) Date of publication of application: **02.03.2022**
(21) Application number: 21191011.2
(22) Date of filing: 12.08.2021
(51) Int. Cl.: A61B 18/12, A61B 18/14, A61B 18/00

(54) **APPLYING BIPOLAR ABLATION ENERGY BETWEEN SHORTED ELECTRODE GROUPS**

(30) Priority: 13.08.2020 US 202016993092
(71) Applicant: Biosense Webster (Israel) Ltd, Yokneam, 2066717 (IL)
(72) Inventor: Altmann, Andres Claudio, Yokneam (IL); Govari, Assaf, Yokneam (IL)
(74) Representative: Carpmaels & Ransford LLP

(57) **Abstract**

A system includes a catheter, a switching assembly, and a processor. The catheter including an expandable frame, which is coupled to a distal end of the catheter, and multiple electrodes, which are disposed on the expandable frame in a radial geometry. The switching assembly is electrically connected to the catheter, and is configured to electrically short between selected ones of the electrodes. The processor is configured, for first and second disjoint groups of the electrodes, to control the switching assembly to electrically short the electrodes within each of the first and second groups, for applying one or more bipolar ablation energy between the first and second groups when the electrodes are placed in contact with a target tissue of the organ.

## Description

### FIELD OF THE INVENTION

The present invention relates generally to tissue ablation, and particularly to methods and systems for performing ablation procedures using an expandable catheter with various energy modalities (RF or IRE).

### BACKGROUND OF THE INVENTION

Various types of catheters, such as balloon catheters, may be used in treatment applications, such as in ablation of a patient organ.

For example, U.S. Patent Application Publication 2020/0155224 describes an expandable balloon, which is coupled to a distal end of a shaft for insertion into an organ of a patient. The balloon includes an expandable membrane, one or more electrodes and one or more respective conductive coils. The one or more electrodes are disposed over an external surface of the membrane. The one or more respective conductive coils are each disposed proximate a respective RF ablation electrode.

U.S. Patent Application Publication 2019/0298441 describes an electrophysiology catheter having a balloon with a membrane. Electrodes may be disposed on the membrane. Each electrode may include a radiopaque marker. The markers may have different forms, e.g., alphanumeric or polygonal, to facilitate visualization of the electrodes using a bi-stable image and allow for selection of the appropriate electrodes to be energized during ablation of tissue.

### SUMMARY OF THE INVENTION

An embodiment of the present invention that is described herein provides a system including a catheter, a switching assembly, and a processor. The catheter including an expandable frame, which is coupled to a distal end of the catheter, and multiple electrodes, which are disposed on the expandable frame in a radial geometry. The switching assembly is electrically connected to the catheter, and is configured to electrically short between selected ones of the electrodes. The processor is configured, for first and second disjoint groups of the electrodes, to control the switching assembly to electrically short the electrodes within each of the first and second groups, for applying one or more bipolar ablation pulses between the first and second groups when the electrodes are placed in contact with a target tissue of the organ.

In some embodiments, at least one of the electrodes is disposed along an axis of the catheter. In other embodiments, the switching assembly includes a mechanical switch or an electronic switch. In yet other embodiments, the electrodes are disposed between a vertex of the expandable frame and a coupling point between the expandable frame and the distal end of the catheter.

In an embodiment, the expandable frame includes a balloon or a basket. In another embodiment, when placed in contact with the target tissue, the electrodes are configured to sense electrical signals from the organ.

In some embodiments, the patient organ includes a patient heart, and the sensed electrical signals include intra-cardiac electrical signals for mapping the patient heart. In other embodiments, the one or more bipolar ablation energy include one or more irreversible electroporation (IRE) pulses.

In an embodiment, the system includes a pulse generator, which is configured to produce at least (i) a first set of the one or more bipolar ablation pulses, and (ii) a second set of the one or more bipolar ablation pulses, different from the first set. In another embodiment, for third and fourth groups of the electrodes that are disjoint from one another and from the first and second groups, the processor is configured to: (i) control the switching assembly to electrically short the electrodes within each of the third and fourth groups, and (ii) control the pulse generator to apply: (a) the first set between the first and second groups, and (b) the second set between the third and fourth groups.

There is additionally provided, in accordance with an embodiment of the present invention, a method including inserting into a patient organ, a catheter including: (i) an expandable frame, coupled to a distal end of the catheter, and (ii) multiple electrodes, which are disposed on the expandable frame in a radial geometry. At least some of the electrodes are placed in contact with a target tissue of the organ. For first and second disjoint groups of the electrodes, the electrodes within each of the first and second groups are electrically shorted therebetween. One or more bipolar ablation pulses are applied between the first and second groups when the electrodes are placed in contact with the target tissue.

In some embodiments, the organ includes heart, and applying the one or more bipolar ablation pulses includes treating arrhythmia in the heart by applying the one or more bipolar ablation pulses to the target tissue of the heart.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based position-tracking and energy generator system 20, in accordance with an embodiment of the present invention;
Fig. 2A is a schematic, side view of a catheter tip of the ablation system, in accordance with an embodiment of the present invention;
Fig. 2B is a schematic, axial view of the catheter tip of the ablation system, in accordance with an embodiment of the present invention;
Fig. 3 is a flow chart that schematically illustrates a method for applying irreversible electroporation (IRE) pulses to tissue using the catheter tip comprising a balloon and multiple electrodes arranged in a radial geometry, in accordance with an embodiment of the present invention;
Fig. 4 is a table showing permutations of number of a single bipolar group of electrodes, in accordance with an embodiment of the present invention, in accordance with an embodiment of the present invention; and
Fig. 5 is a table showing permutations of number of dual bipolar groups of electrodes, in accordance with another embodiment of the present invention.

### DETAILED DESCRIPTION OF EMBODIMENTS

### OVERVIEW

Irreversible electroporation (IRE) may be used, for example, for treating arrhythmia by ablating tissue cells using high-voltage applied pulses. Cellular destruction occurs when the transmembrane potential exceeds a threshold, leading to cell death and formation of a lesion. In IRE-based ablation procedures, high-voltage bipolar electrical pulses are applied, for example, to a pair of electrodes in contact with tissue to be ablated, so as to form a lesion between the electrodes, and thereby to treat arrhythmia in a patient heart.

Embodiments of the present invention that are described hereinbelow provide improved techniques for applying one or more IRE pulses to a target tissue in patient heart, such an ostium of a pulmonary vein (PV).

In some embodiments, a system configured to perform IRE ablation procedures comprises an ablation catheter having an expandable frame, such as a balloon, coupled to a distal end of the catheter. The balloon is configured for insertion into an ablation site, such as the PV ostium, having tissue intended to be ablated.

In some embodiments, the ablation catheter comprises multiple electrodes, which are disposed on the balloon in a radial geometry, and are placed, by a physician, in contact with the target tissue at the ablation site.

In some embodiments, the system comprises a switching assembly, which is electrically connected to the catheter, and is configured to electrically short between selected ones of the electrodes disposed on the balloon.

In some embodiments, the system comprises a processor, which is configured, for first and second disjoint groups of the electrodes, to control the switching assembly to electrically short the electrodes within each of the first and second groups. The processor is further configured to control a radiofrequency (RF) generator for applying one or more bipolar ablation energy between the first and second groups when the electrodes are placed in contact with the target tissue of the patient heart.

The disclosed techniques enable applying IRE pulses to target tissue using a catheter having a relatively small balloon or basket (e.g., expandable to about 10 mm) and a small inter-electrode distance.

### SYSTEM DESCRIPTION

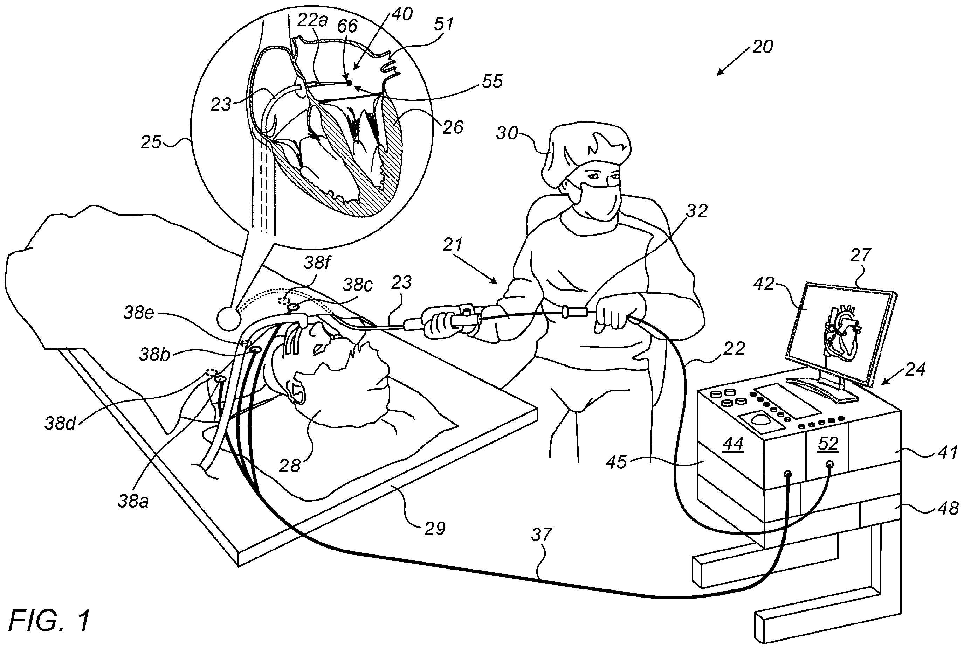

Fig. 1 is a schematic, pictorial illustration of a catheter-based position-tracking and energy generator system 20, in accordance with an embodiment of the present invention.

Reference is now made to an inset 25. In some embodiments, system 20 comprises a catheter tip 40 that is fitted at a distal end 22a of a shaft 22 of a catheter 21 shown in the general view of Fig. 1.

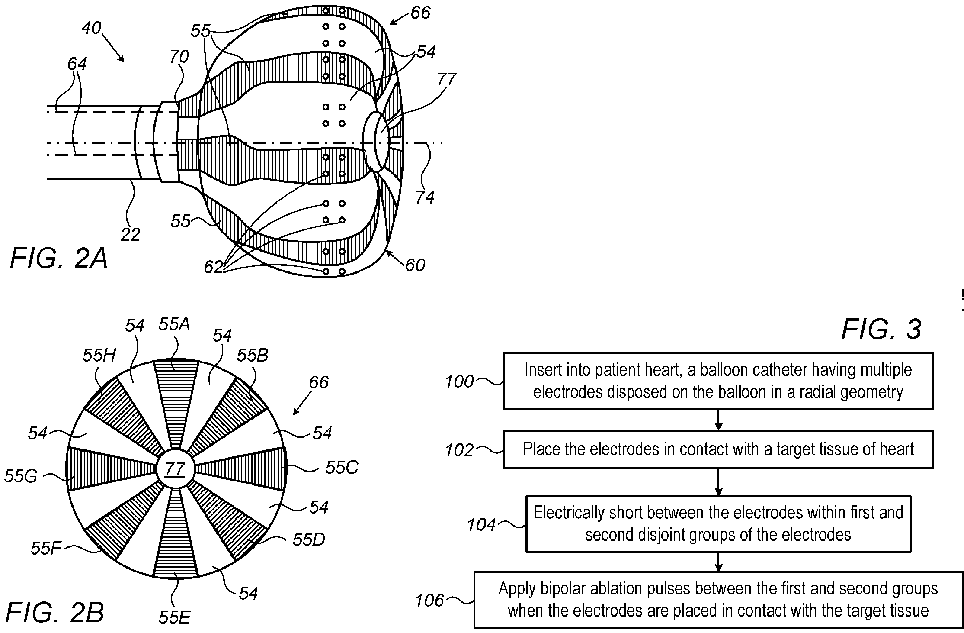

In some embodiments, catheter tip 40 comprises an expandable frame described in detail if Figs. 2A and 2B below. In the present example, the expandable frame comprises an inflatable balloon 66 having multiple electrodes, such as but not limited to multiple RF ablation electrodes 55. In some embodiments, electrodes 55 are configured to sense bipolar intra-cardiac signal from an ostium 51 of a pulmonary vein (PV) in a heart 26 of a patient 28. Moreover, electrodes 55 are configured for ablating one or more bipolar ablation pulses to ostium 51 in heart 26. In the present example, the one or more bipolar ablation energy comprise irreversible electroporation (IRE) pulses described in detail below.

IRE, also referred to as Pulsed Field Ablation (PFA), may be used as a minimally invasive therapeutic modality to kill tissue cells at the ablation site by applying high-voltage pulses to the tissue. In the present example, IRE pulses may be used for killing myocardium tissue cells in order to treat cardiac arrhythmia in heart 26. Cellular destruction occurs when the transmembrane potential exceeds a threshold, leading to cell death and thus the development of a tissue lesion. Applying IRE pulses to tissue during a bipolar ablation procedure is described in detail below.

Reference is now made back to the general view of Fig. 1. In some embodiments, the proximal end of catheter 21 is connected to a control console 24 comprising a pulse generator, in the present example, apulse (or RF) generator 45 for applying the IRE pulses to tissue of ostium 51. An ablation protocol comprising ablation parameters is stored in a memory 48 of console 24. As used herein, the bipolar ablation energy can be in the form of "IRE" or "IRE pulses" refers to discrete non-ramping or step-wise pulses of certain durations or in the form of "RF" signals having discrete step-wise ramps of alternating waveform or continuously alternating radio-frequency waveform.

In some embodiments, a physician 30 inserts distal end 22a of shaft 22 through a sheath 23 into heart 26 of patient 28 lying on a table 29. Physician 30 advances the distal end of shaft 22 to ostium 51, also referred to herein as a target tissue, in heart 26 by manipulating shaft 22 using a manipulator 32 near the proximal end of catheter 21. During the insertion of distal end 22a, catheter tip 40 is maintained inside sheath 23 so as to minimize vascular trauma along the way to target tissue.

In some embodiments, system 20 comprises an impedance-based active current location (ACL) system, which may be used by physician 30 for navigating and tracking the position of catheter tip 30 in heart 26.

In an embodiment, physician 30 navigates the distal-end of shaft 22 to the target tissue by tracking the position of catheter tip 40. During navigation of distal end 22a in heart 26, console 24 receives signals from a coil (not shown) or from any other element, e.g., any of electrodes 55, which is configured to serve as an impedance-based position sensor of the ACL system.

In some embodiments, the ACL system comprises a plurality of electrodes 38, which are coupled to the body of patient 28, e.g., via patches 29 that adhere to the skin of patient 28. In the example of Fig. 1, system 20 comprises six electrodes, of which electrodes 38a, 38b, and 38c are coupled to the front (e.g., chest) of patient 28, and electrodes 38d, 38e, and 38f are coupled to the back of patient 28. As shown in Fig. 1, the electrodes are arranged in pairs as follows: electrodes 38a and 38d are facing one another at the right side of patient 28, electrodes 38c and 38f are facing one another at the left side of patient 28, and electrodes 38b and 38e are facing one another at the upper part of the chest and back of patient 28.

In other embodiments, system 20 may comprise any suitable number of electrodes, coupled to the patient skin in any suitable arrangement.

In some embodiments, electrodes 38a-38f are typically connected, via a cable 37, to a processor 41 of system 20, which is configured to receive from electrodes 38a-38f electrical signals indicative of the measured impedance, and, based on the received signals, to estimate the position of catheter tip 40 within heart 26 using techniques described herein.

In some embodiments, electrodes 38a-38f are typically used for navigating catheter 21 within the body of patient 28, using the aforementioned impedance-based ACL system and tracking techniques, such as those described, for example, in US Patent 8,456,182 and US Patent Application Publication 2015/0141798, whose disclosures are incorporated herein by reference.

In some embodiments, the ACL system is configured to estimate the position of catheter tip 40 responsively to the different impedances measured between the aforementioned coil or electrode 55 coupled to catheter tip 40, and each of electrodes 38a-38f.

In some embodiments, processor 41 is configured to estimate the position of catheter tip 40 in heart 26, and to display on a display 27 of console 24, a marker (not shown) overlaid on an anatomical image 42 (or a synthetic model) of heart 26. Physician 30 may use the marker, e.g., for navigating catheter tip 40 into ostium 51.

In some embodiments, once distal end 22a of shaft 22 has reached heart 26, physician 30 retracts sheath 23 and further manipulates shaft 22 to navigate catheter tip 40 to ostium 51 of the pulmonary vein, or to any other target tissue of heart 26.

In some embodiments, system 20 comprises a switching assembly 52, which is electrically connected to catheter 21, and is configured to electrically short between selected electrodes 55 of catheter tip 40. In the present example, switching assembly 52 is implemented in console 24 and may comprise a mechanical switch, an electronic switch, a combination thereof, or any other suitable type of switch. Note that the selected electrodes 55 are shorted in two disjoint groups, referred to herein as first and second groups.

In some embodiments, processor 41 is configured, for the first and second disjoint groups of electrodes 55, to control switching assembly 52 to electrically short electrodes 55 within each of the first and second groups. Processor 41 is further configured to control power generator 45 to apply the IRE pulses, via catheter 21, between the first and second groups when the selected electrodes 55 are placed in contact with the target tissue of heart 26.

In some embodiments, while catheter tip 40 is placed in contact with the tissue, physician 30 may control system 20 for acquiring bipolar intra-cardiac electrical signals from the target tissue of heart 26, for example, using electrodes 55 in contact with the target tissue.

Processor 41 is typically a general-purpose computer, with suitable front end and interface circuits 44 for receiving signals from catheter 21 and ECG signals from electrodes 38, and for controlling the other components of system 20. Processor 41 typically comprises a software in a memory 48 of system 20 that is programmed to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by embodiments of the present invention and to demonstrate the application of these embodiments in enhancing the performance of such an ablation system. Embodiments of the present invention, however, are by no means limited to this specific sort of example system, and the principles described herein may similarly be applied to other sorts of ablation systems.

In other embodiments, instead of balloon 66, catheter tip 40 may have any other suitable component, such as an expandable basket or any other suitable type of an expandable frame.

### PERFORMING BIPOLAR ABLATION BY SHORTING BETWEEN SELECTED ELECTRODES OF ABLATION BALLOON CATHETER

Fig. 2A is a schematic, side view of catheter tip 40, in accordance with an embodiment of the present invention.

In some embodiments, catheter tip 40 comprises balloon 66 coupled to distal end 22a of shaft 22. Balloon 66 comprises a membrane 60, which is expandable and having irrigation holes 62 for irrigating the target tissue in the ablation procedure. The configuration of irrigation holes 62 is provided by way of example, and in other embodiments, balloon 66 may have any suitable number of irrigation holes 62 formed in any suitable positions on the surface of balloon 66.

In some embodiments, balloon 66 comprises electrodes 55 disposed along an axis 74 of balloon 66, electrodes 55 are disposed on the surface of membrane 60 in a radial geometry, as will be shown more clearly in Fig. 2B below. Typically, each electrode 55 of catheter 21 is electrically connected to switching assembly 52, via one or more electrical wires 64 running along catheter 21.

In some embodiments, electrodes 55 are disposed, along axis 74, between a vertex 77 of balloon 66 and a coupling point 70 between electrodes 55 and electrical wires 64. Note that both vertex 77 and coupling point 70 are electrically insulating between electrodes 55.

In the example of Fig. 2A, balloon 66 has bare areas 54 positioned alternately between electrodes 55, so as to electrically insulate between adjacent electrodes 55. In other embodiments, balloon 66 may have aby other suitable configuration for electrically insulating between adjacent electrodes 55.

As described in Fig. 1 above, cellular destruction occurs when the transmembrane potential exceeds a threshold, leading to cell death and thus the development of a tissue lesion. However, balloon 66 has a diameter between about 9 mm and 12 mm, and therefore, electrodes 55, specifically 55A, 55B, 55C, 55D, 55E, 55F, 55H, are separated by relatively short distances (e.g., bare or non-conductive separation areas 54), which is a problem when electrodes 55 are used for bipolar ablation of ostium 51, because the effective region of each electrode 55 may be insufficient to form the desired lesion in ostium 51 by applying bipolar ablation energy (whether by IRE pulses or RF bipolar energy).

In the context of the present disclosure and in the claims, the terms "about" or "approximately" for any numerical values or ranges indicate a suitable dimensional tolerance that allows the part or collection of components to function for its intended purpose as described herein.

In some embodiments, applying high-voltage bipolar electrical energy (i.e., by pulses for IRE or waves for RF) may be carried out using two groups of electrodes 55 in contact with tissue at the ablation site. Using the aforementioned first and second groups may produce sufficiently large area of each group of electrodes (e.g., about 10 mm2), and a sufficiently large (e.g., about 15 mm²) bare area 54 between the groups, so as to generate high electric fields (e.g., above a certain threshold) to kill tissue cells located between the first and second groups. Hence, a switching assembly can be configured to electrically short between selected ones of the electrodes so that a combined surface area of the selected electrodes is at least approximately 10 squared millimeters and a combined separation areas are at least approximately 15 squared millimeters. And as used herein, the term "bare area" means an area with no electrode or conductive surface, i.e., a non-conductive separation area between any two adjacent electrodes.

In the context of this disclosure, "bipolar" voltage pulse means a discrete non-ramping type voltage pulse applied between the two or more disjoint groups of electrodes 55 of catheter 21 (as opposed, for example, to alternating gradual radiofrequency wave signals that are applied, e.g., during a radiofrequency ablation, by a catheter electrode relative to some common ground electrode not located on the catheter or inside the subject body) and hence this technique is referenced as "unipolar" ablation. That is, in the context of bipolar IRE ablation, one or more pulses of a first polarity (e.g., positive pole) are applied to a first group of electrodes (or second group of electrodes) and one or more pulses of a second polarity (e.g., negative pole) are applied to the second group of electrodes (or first group of electrodes). The term "pulse" means that the voltage is switched very quickly (without any appreciable step wise or continuous ramping) between the polarities such that there is effectively no gradual ramping from one polarity to the other polarity such as, for example, in a sine wave. In the context of RF bipolar ablation, RF waveform (e.g., sinewave) of a predetermined frequency are applied between any two or more groups of electrodes defining a bipole set of electrodes.

To implement IRE ablation over a relatively large tissue region of heart 26, such as a circumference of ostium 51 of the aforementioned PV or any other suitable organ, it is necessary to use in at least one of the groups, multiple electrodes 55, and sufficiently large bare area 54 separating between the first and second groups.

In some embodiments, switching assembly 52 is configured to electrically short between selected ones of electrodes 55. As described in Fig. 1 above, processor 41 is configured, for the first and second disjoint groups of electrodes 55, to control switching assembly 52 to electrically short electrodes 55 within each of the first and second groups. Examples of the disjoint groups are described in Fig. 2B below.

In some embodiments, when at least some electrodes 55 are placed in contact with the target tissue of heart 26, processor 41 is configured to control power generator 45 for applying one or more IRE pulses (or any other type of bipolar ablation energies such as radio-frequency or RF), between the first and second groups of electrodes 55.

Fig. 2B is a schematic, axial view of balloon 66 of catheter tip 40, in accordance with an embodiment of the present invention. In some embodiments, balloon 66 comprises eight electrodes 55, referred to herein as electrodes 55A, 55B, 55C, 55D, 55E, 55F, 55G, and 55H, arranged in a radial geometry with non-conductive separation areas 54 between any two adjacent electrodes.

In some embodiments, processor 41 is configured to control switching assembly 52 to short selected electrodes 55 within each of the first and second groups. For example, processor 41 is configured to select, and control switching assembly 52 to short between (i) electrodes 55A and 55C for the first group, and (ii) electrodes 55B and 55D for the second group. In this example, the target tissue in contact with balloon 66 between electrodes 55A and 55D will be ablated when processor 41 controls power generator 45 to apply the IRE pulses.

In another example, processor 41 is configured to select, and control switching assembly 52 to short between (i) electrodes 55H, 55A and 55B for the first group of bipole electrode, and (ii) electrodes 55D and 55E for the second group of the other bipole electrode (and together the first and second groups define a bipole set of electrodes. In this example, the target tissue in contact with balloon 66 between electrodes 55B and 55D, and between electrodes 55H and 55E will be ablated when processor 41 controls power generator 45 to apply the IRE pulses.

In some embodiments, in the configuration of Fig. 2B, each of the first and second groups may comprise between one and seven electrodes. For example, the first group may comprise electrode 55A and the second group may comprise electrodes 55B and 55H, so as to ablate the target tissue in contact between electrodes 55B and 55H. Note that the first and second groups are disjoint, so that different electrodes 55 are selected for the different first and second groups. In other words, the term "disjoint" as applied to electrodes means that no electrode from the plurality of electrodes 55A through 55H may be selected for both the first and second groups at the same time.

In some embodiments, the selection of electrodes 55 for the first and second groups may be carried out based on instructions from physician 30. In other embodiments, the selection of electrodes 55 for the first and second groups may be carried out autonomously, e.g., by processor 41, after mapping which electrodes 55 are in contact with the target tissue, e.g., by receiving from the respective electrodes 55 intra-cardiac signals sensed in the target tissue.

In alternative embodiments, processor 41 may receive the intra-cardiac signals from electrodes 55 in contact with the target tissue, and may recommend to physician 30, e.g., based on a predefined ablation plan, electrodes 55 to be selected for the first and second groups. Moreover, based on the intra-cardiac signals received from electrodes 55, processor 41 may identify electrodes having insufficient contact with the target tissue. For example, in a PV-isolation ablation procedure applied to ostium 51, processor 41 may detect, based on intra-cardiac signals received from electrodes 55, or based on signals received from a contact-force sensor (not shown) indicative of insufficient contact force between one or more electrodes 55 and the target tissue of ostium 51. In such embodiments, processor 41 may recommend physician 30 to adjust the position and inflation-level of balloon 66, so that all electrodes 55 are in sufficient contact force with the target tissue of ostium 51.

The configuration of balloon 66, as shown in Figs. 2A and 2B is provided by way of example, in order to illustrate certain problems that are addressed by embodiments of the present invention and to demonstrate the application of these embodiments in enhancing the performance of a catheter tip of such an ablation catheter. Embodiments of the present invention, however, are by no means limited to this specific sort of example catheter tip, and the principles described herein may similarly be applied to other sorts of ablation catheters.

Fig. 3 is a flow chart that schematically illustrates a method for applying IRE pulses to tissue using balloon 66 and multiple electrodes 55 arranged in a radial geometry, in accordance with an embodiment of the present invention.

The method begins at a catheter insertion step 100, with inserting into patient heart 26, balloon 66 of catheter tip 40 having multiple electrodes 55 disposed on balloon 66 in a radial geometry. At an electrode placing step 102, at least some of electrodes 55 are placed in contact with the target tissue (e.g., at ostium 51) of heart 26, as described in detail in Figs. 1, 2A and 2B above.

At a selected electrodes shorting step 104, processor 41 controls switching assembly 52 to electrically short between selected electrodes 55 within the first and second disjoint groups of electrodes 55, as described in detail in Figs. 1, 2A and 2B above.

At a bipolar ablation step 106, which concludes the method and bipolar ablation, processor 41 controls power generator 45 to apply bipolar ablation energy between the first and second groups when the selected electrodes of the first and second groups are placed in contact with the target tissue, as described in detail in Figs. 1, 2A and 2B above.

After concluding the bipolar ablation, physician 30 may extract catheter tip 40 out of the body of patient 28.

Although the embodiments described herein in one exemplary embodiment for that of irreversible electroporation (IRE) ablation procedures, the methods and systems described herein can also be used in other applications, such as in any sort of bipolar RF ablation as well as electrosurgical procedures.

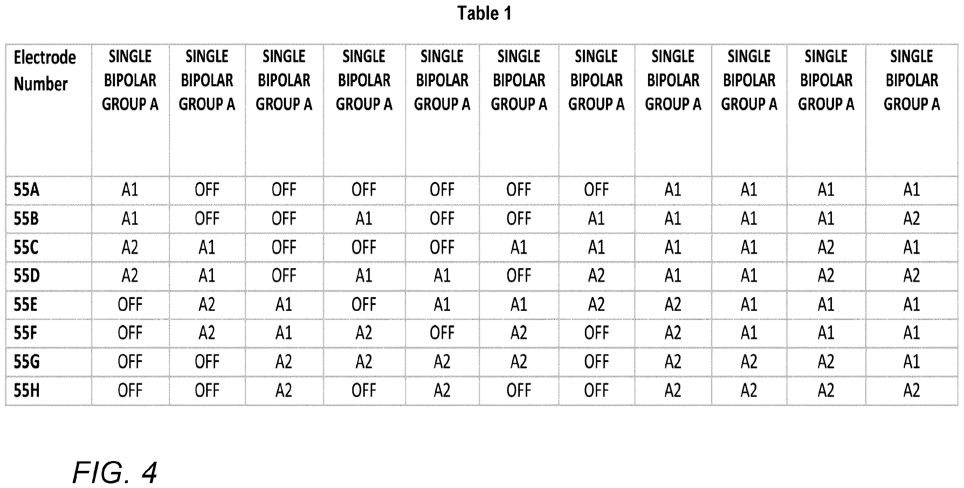

Fig. 4 is a table 1 showing permutations of number of a single bipolar group of electrodes 55, in accordance with an embodiment of the present invention. In table 1, each column refers to an example of a bipolar group, and the terms "A1" and "A2" refer to first and second respective groups of electrodes 55 applying energy to tissue. In the first example, electrodes 55A and 55B constitute a first group of electrodes denoted "A1," and electrodes 55C and 55D constitute a second group of electrodes denoted "A2." In this example, electrodes 55E, 55F, 55G and 55H are not applying energy to tissue, and therefore, are denoted "OFF."

In some embodiments, processor 41 may apply pulses to all electrodes 55 as described in Fig. 2B above. For example, as shown in the last (i.e., right-most) column of table 1, electrodes 55A, 55C, 55E, 55F and 55G are marked in table 1 with group "A1" (55A,55C,55E,55F,55G) and constitute the first group of combined bipole electrodes, and electrodes 55B, 55D and 55H are marked in table 1 with "A2" (55B,55D,55H) and constitute the second group of combined bipole electrodes. In effect, the combination of electrodes in group A1 constitutes a first large bipole electrode and the combined electrodes in group A2 constitutes another large (or second large) bipole electrode and together group A1 and group A2 define the two poles for bipolar ablation. It is noted that while the examples show at least two electrodes per group, it is within the scope of the invention to have only one electrode in one bipole group (e.g., 55A in A1) and two or more electrodes in the other bipole group (e.g., 55B,55C,55D,55E,55F,55G,55H for A2).

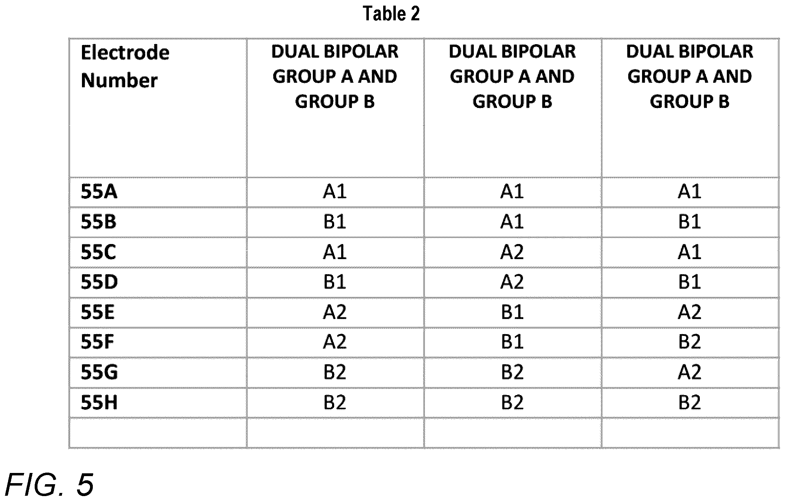

Fig. 5 is a table 2 showing permutations of number of dual bipolar groups of electrodes, in accordance with another embodiment of the present invention. In some embodiments, processor 41 is configured to control power generator 45 to apply different sets of one or more bipolar ablation energy to different groups of electrodes 55. The sets may differ in voltage, and/or in frequency, and/or in any other attribute of the energy. In table 2, each column refers to an example of two bipolar groups "A" and "B." The terms "A1" and "A2" refer to first and second respective groups of electrodes 55 applying a first set of bipolar energy to tissue, and the terms "B1" and "B2" refer to third and fourth respective groups of electrodes 55 applying a second set of bipolar energy to tissue.

For example, as shown in the right-most column of table 2, (i) electrodes 55A and 55B, marked with "A1," constitute the first group of bipole electrode, and (ii) electrodes 55E and 55G, marked with "A2," constitute the second group of bipole electrode. Both first and second groups A1 and A2 define a first set of bipolar electrodes to apply the first set of bipolar energy to tissue. Group A1 and A2 can be configured to deliver a first magnitude of energy for a first duration. Similarly, (i) electrodes 55B and 55D, marked with "B1," constitute the third group of another bipole electrode, and (ii) electrodes 55F and 55H, marked with "B2," constitute the fourth group of electrodes 55 for a counterpart pole to the group B1. Both third and fourth groups B1 and B2 define a second set of bipolar electrodes to apply the second set of bipolar energy to tissue. That is, bipolar electrodes B1 and B2 can be configured to deliver a second magnitude of energy (different from the first magnitude of bipole electrode A1 and A2) at a second duration (different from the first duration of bipole electrode A1 and A2). Stated in another way, the system can be configured to provide: (i) a first set of the one or more bipolar ablation pulses to a first group of bipole electrodes (A1 and A2), and (ii) a second set of the one or more bipolar ablation pulses, different from the first set to a second group of bipole electrodes (B1 and B2). As in the examples in Fig. 4, it is within the scope of the invention to have only one electrode in group A1 (or A2) and two or more electrodes in group A2 (or A1) as well as only one electrode in group B1 (or B2) and two or more electrodes in group B2 (or B1).

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method, comprising:
   inserting into a patient organ, a catheter comprising: (i) an expandable frame, coupled to a distal end of the catheter, and (ii) multiple electrodes, which are disposed on the expandable frame in a radial geometry;
   placing at least some of the electrodes in contact with a target tissue of the organ;
   for first and second disjoint groups of the electrodes, electrically shorting between the electrodes within each of the first and second groups; and
   applying one or more bipolar ablation energy between the first and second groups when the electrodes are placed in contact with the target tissue.
2. The method according to aspect 1, wherein at least one of the electrodes is disposed along an axis of the catheter.
3. The method according to aspect 1, wherein the electrodes are disposed between a vertex of the expandable frame and a coupling point between the expandable frame and the distal end of the catheter.
4. The method according to aspect 1, wherein the expandable frame comprises a balloon or a basket.
5. The method according to aspect 1, and comprising sensing electrical signals from the organ.
6. The method according to aspect 5, wherein the patient organ comprises a patient heart, and wherein sensing the electrical signals comprises sensing intra-cardiac electrical signals for mapping the patient heart.
7. The method according to aspect 1, wherein applying the one or more bipolar ablation energy comprises applying, to the target tissue, one or more irreversible electroporation (IRE) pulses.
8. The method according to aspect 1, and comprising producing at least (i) a first set of the one or more bipolar ablation energy, and (ii) a second set of the one or more bipolar ablation energy, different from the first set.
9. The method according to aspect 8, wherein, for third and fourth groups of the electrodes that are disjoint from one another and from the first and second groups, (i) electrically shorting the electrodes within each of the third and fourth groups, and (ii) applying, (a) the first set between the first and second groups, and (b) the second set between the third and fourth groups.
10. The method according to aspect 1, wherein the organ comprises heart, and wherein applying the one or more bipolar ablation energy comprises treating arrhythmia in the heart by applying the one or more bipolar ablation energy to the target tissue of the heart.

## Claims

1. A system, comprising:
a catheter comprising:
an expandable frame, coupled to a distal end of the catheter; and
multiple electrodes, which are disposed on the expandable frame in a radial geometry;
a switching assembly, which is electrically connected to the catheter, and is configured to electrically short between selected ones of the electrodes; and
a processor, which is configured, for first and second disjoint groups of the electrodes, to control the switching assembly to electrically short the electrodes within each of the first and second groups, to apply one or more bipolar ablation energy between the first and second groups when the electrodes are placed in contact with a target biological tissue.

2. The system according to claim 1, wherein at least one of the electrodes is disposed along an axis of the catheter.

3. The system according to claim 1, wherein the switching assembly comprises a mechanical switch or an electronic switch.

4. The system according to claim 1, wherein the electrodes are disposed between a vertex of the expandable frame and a coupling point between the expandable frame and the distal end of the catheter.

5. The system according to claim 1, wherein the expandable frame comprises a balloon or a basket.

6. The system according to claim 1, wherein, when placed in contact with the target tissue, the electrodes are configured to sense electrical signals from the organ.

7. The system according to claim 6, wherein the patient organ comprises a patient heart, and wherein the sensed electrical signals comprise intra-cardiac electrical signals for mapping the patient heart.

8. The system according to claim 1, wherein the one or more bipolar ablation energy comprises one or more irreversible electroporation (IRE) pulses.

9. The system according to claim 1, and comprising a pulse generator, which is configured to produce at least (i) a first set of the one or more bipolar ablation pulses to a first group of bipole electrodes, and (ii) a second set of the one or more bipolar ablation pulses, different from the first set to a second group of bipole electrodes.

10. The system according to claim 9, wherein, for third and fourth groups of the electrodes that are disjoint from one another and from the first and second groups, the processor is configured to: (i) control the switching assembly to electrically short the electrodes within each of the third and fourth groups, and (ii) control the pulse generator to apply: (a) the first set between the first and second groups, and (b) the second set between the third and fourth groups.
